# EUROPEAN PATENT SPECIFICATION

(11) **EP 1 331 218 B1**
(45) Date of publication and mention of the grant of the patent: **27.12.2006**
(21) Application number: 03006773.0
(22) Date of filing: 28.05.1999
(51) Int. Cl.: C07C 209/00, C07C 29/62

(54) **Process for the preparation of protected 3-amino-1,2-dihydroxy-propane acetal and derivatives thereof**
Verfahren zur Herstellung von geschützten 3-Amino-1,2-Dihydroxypropanacetal und Derivate davon
Procédé de préparation du 3-amino-1,2-dihydroxypropane acétal protégé et de ses dérivés

(30) Priority: 01.06.1998 US 87496 P
(43) Date of publication of application: 30.07.2003
(62) Divisional of application: 99955263.1
(73) Proprietor: Michigan State University, East Lansing, Michigan 48824 (US)
(72) Inventor: Hollingsworth, Rawle I., Haslett, Michigan 48840 (US); Wang, Guijun, New Haven, Connecticut 06511 (US)
(74) Representative: Grünecker, Kinkeldey, Stockmair & Schwanhäusser Anwaltssozietät

(56) References cited:
- WO-A-99/52855
- US-A- 4 900 847
- M.M.FAUL ET AL.: "Macrocyclic Bisindolylmaleimides: Synthesis by Inter- and Intramolecular Alkylation" JOURNAL OF ORGANIC CHEMISTRY, vol. 63, 1998, pages 1961-1973, XP000996636
- G. WANG ET AL.: "Direct conversion of (S)-3-Hydroxy-gamma-butyrolactone to ciral three-carbon building blocks" JOURNAL OF ORGANIC CHEMISTRY, vol. 64, no. 3, 1999, pages 1036-1038, XP002242435
- DANKLMAIER J. ET AL: 'Synthese and struktur diastereomerenreiner 2,6-disubsituierter 3-Morpholinone' LIEBIGS ANN.CHEM. 1988, pages 1149 - 1153, XP000986227
- SOWDEN J.C. ET AL: 'l-Glycidol' J.AM.CHEM.SOC. 1942, pages 1291 - 1293
- FURROW M.E. ET AL: 'Practical access to highly enantioenriched C-3 building blocks via hydrolytic kinetic resolution.' J.ORG.CHEM. vol. 63, 1998, pages 6776 - 6777

## Description

The present invention relates to a process for the preparation of protected 3-amino-1,2-dihydroxypropane acetal, particularly in chiral forms, for use as an intermediate in the preparation of various 3-carbon compounds which are chiral. In particular, the present invention relates to the process for preparation of 3-amino-1,2-dihydroxypropane isopropylidene acetal. The invention is particularly useful for preparation of glycidol, 1-bromo-2,3-dihydroxypropane, or 3-amino-1,2-dihydroxypropane starting from 3-hydroxy-γ-butyrolactone.

Chiral 3-carbon synthons are very important compounds because they are used in a variety of pharmaceuticals and material science applications ranging from beta-blocker drugs, phospholipid and glycolipid analogs, thromboxane synthase inhibitors, sulfolipids and liquid crystal materials. However, chiral 3-carbon synthons are extremely expensive. Three key 3-carbon building blocks are (R)-glycidol **(2),** (R)-1-bromo-2,3-dihydroxypropane **(3)** and (S)-3-amino-1,2-dihydroxypropane **(4).**

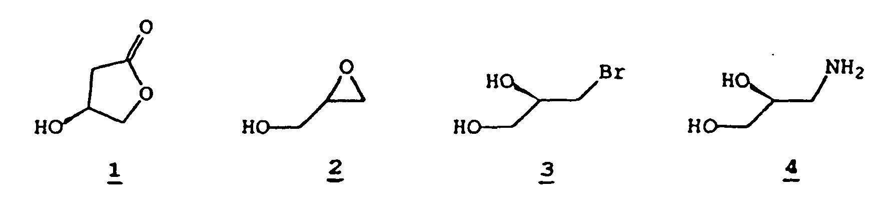

(R)-glycidol **(2)** and its (S)-isomer are much used intermediates in the synthesis of chiral compounds. Because of this, much effort has been expended in developing routes to them. These include catalytic oxidations with peroxides and chiral transition metal complexes (Byun, H.-S, et al., J. Org. Chem. **59**:668-671 (1994)), enzymatic resolutions of racemic esters using lipases to selectively deacylate one member enantiomer (Bednarski, M.D., et al., J. Am. Chem. Soc. **109**:1283-1285 (1987); Fuganti, C., et al., Tetrahedron **44**:2575-2582 (1988); and Chen, J., et al., Tetrahedron Lett. **34**:7663-7666 (1993)), and treatment of a chiral 1,2-propane diol with a leaving group such as a halide or tosylate ester in the 3-position with base (Baldwin, J. J., et al., J. Med. Chem. **25**:931-936 (1982)). The availability of an easy route to **3** is therefore *de facto* a route to **2.** The aminodiol **4** is a substructure that appears in a large class of important drugs especially the β-blockers such as Propanalol **(5)** and Metoprolol **(6)**, the antiviral agent **(7)** and the thromboxane synthase inhibitor **(8)**.

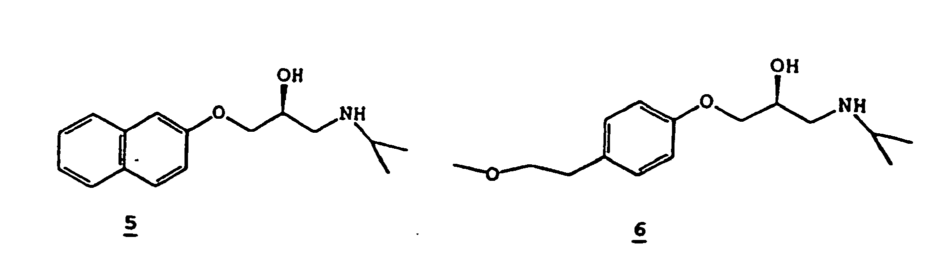

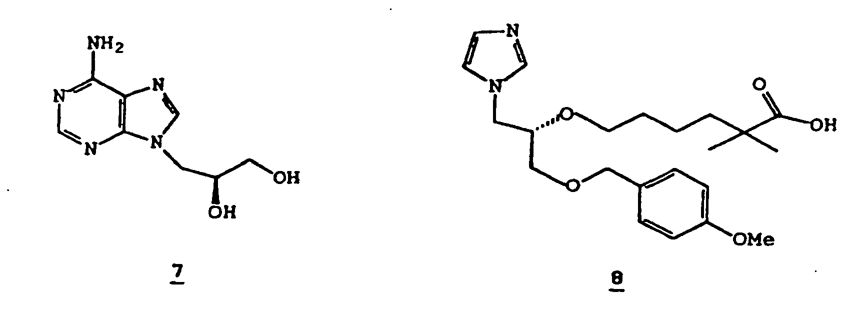

The problem underlying the present invention is to provide a process for preparing 3-carbon synthons, in particular in chiral form, which is inexpensive, safe, and easy to perform.

This problem is solved by a process for preparing a protected 3-amino-1,2-dihydroxy-propane acetal in a Hoffman rearrangement reaction mixture, having the formula (1): 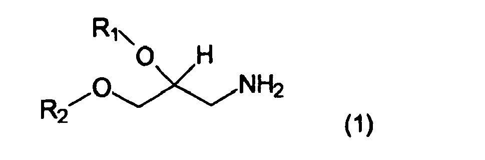 wherein R₁ and R₂ are protecting groups which can be combined
which comprises
reacting a protected 1,2-dihydroxybutryamide in a reaction mixture, having the formula (2): 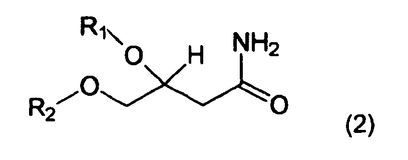 wherein R₁ and R₂ are the same protecting groups
with a hypohalite in the presence of a base in a solvent for the reaction mixture and recovering the protected 3-amino-1,2-dihydroxypropane acetal by solvent extraction.

Preferred embodiments are set forth in the subclaims.

The present invention relates thus to a process for preparing a protected 3-amino-1,2-dihydroxypropane acetal in a Hoffman rearrangement reaction mixture, which comprises reacting a 4-carbon protected 1,2-dihydroxybutryamide in a Hoffman rearrangement reaction mixture with a hypohalite in the presence of a base in a solvent for the reaction mixture to produce the 3-carbon protected 3-amino-1,2-dihydroxypropane acetal. The process uses 3-hydroxy-γ-butyrolactone as the starting material. The protected 3-amino-1,2-dihydroxypropane is a useful intermediate for the synthesis of 3-carbon chiral compounds important for the manufacture of pharmaceuticals.

Thus, the present invention provides a process for preparing 3-carbon compounds which are useful for the manufacture of a variety of pharmaceutical compounds. The process uses as the starting material, 3-hydroxy-γ-butyrolactone **(1)** which is a renewable natural resource that is obtainable from starch, lactose, maltodextrins and other readily available carbohydrate feedstock.

In the process of the present invention, the protected 3,4-dihydroxybutyramide is produced from 3,4-dihydroxybutyramide in a reactive mixture which comprises an acid and a protecting group in a solvent for the reaction to produce the protected 3,4-dihydroxybutyramide. Furthermore, in the process the 3,4-dihydroxybutyramide is produced from 3-hydroxy-y-butyrolactone in a reactive mixture comprising ammonia in a solvent for the reaction to produce the protected 3,4-dihydroxybutyramide. In the preferred process the 3-hydroxy-γ-butyrolactone and protected 3-amino-1,2-dihydroxypropane acetal produced are chiral. The process can use protecting groups selected from the group consisting of alkyloxy, aryloxy, acyloxy, halo, sulfonyloxy, sulfate, phosphate, saccharide and combinations thereof. In particular, the protecting group is an acetal selected from the group consisting of alkylidene, arylidene; acylidene and combinations thereof. In a preferred embodiment, the protecting group is a geminal dimethoxy-acetal.

The present invention further relates to the production of 3-carbon intermediates useful manufacture of pharmaceutical compounds. In particular, the production of 3-amino-1,2-dihydroxypropane wherein the protected 3-amino-1,2-dihydroxypropane acetal produced is further reacted to an acid which produces the 1-amino-2,3-dihydroxypropane; the production of 1-halo-2,3-dihydroxypropane wherein the protected 3-amino-1,2-dihydroxypropane acetal produced is further reacted with a halide source and a nitrite in the presence of an acid to produce the 1-halo-2,3-dihydroxypropane; or the production of 2,3-epoxy wherein in addition the 1-halo-2,3-dihydroxypropane is reacted with a base to form chiral 2,3-epoxy-1-hydroxypropane. In the preferred process the protected 3-amino-1,2-dihydroxypropane acetal is chiral and is converted as above to chiral 3-amino-1,2-dihydroxypropane or chiral 1-halo-2,3-dihydroxypropane are chiral. In the process for producing 1-halo-2,3-dihydroxypropane the halo is selected from the group consisting of Cl, Br, I and F.

In a preferred process, the present invention relates to a process which comprises: (a) reacting 3-hydroxy-γ-butyrolactone with ammonia to produce 3,4-dihydroxybutyramide; (b) reacting the 3,4-dihydroxybutyramide with acetone and dimethyoxypropane in the presence of an acid to produce 3,4-dihydroxybutyramide isopropylidene acetal; and (c) reacting the 1,2-dihydroxybutyramide isopropylidene acetal with an hypohalite in the presence of a base to produce 3-amino-1,2-dihydroxypropane isopropylidene acetal.

The process uses chiral 3-hydroxy-γ-butyrolactone as the starting material so that the product will be chiral. The use of (S)-3-hydroxy-γ-butyrolactone **(1)** as the starting material for synthesis of **5** is convenient over prior art methods because **1** can be synthesized in high yield and in large quantities from renewable, natural resources. Inexpensive methods for synthesizing **1** have been described in: U.S. Patent No. 5,319,110 to R. Hollingsworth which discloses a process for synthesis of an internal cyclic ester such as a lactone by converting a hexose source, which contains hexose as a substituent and another sugar attached to the hexose substituent in the 4 position via (S)-3,4-dihydroxybutanoic acid as an intermediate; U.S. Patent No. 5,374,773 to R. Hollingsworth which discloses a process for the synthesis (S)-3,4-dihydroxybutanoic salt by converting a hexose source which contains hexose as a substituent and another sugar attached to the hexose substituent in the 4 position via (S)-3,4-dihydroxybutyric acid as an intermediate; U.S. Patent No. 5,292,939 to R. Hollingsworth which discloses synthesis of (S)-3,4-dihydroxybutyric acid from substituted D-hexose; and U.S. Patent No. 5,808,107 to R. Hollingsworth which discloses another process for producing chiral lactones. These references are herein incorporated by reference.

The general pathway for the synthesis of protected 3-amino-1,2-dihydroxypropane acetal is shown in Scheme 1. 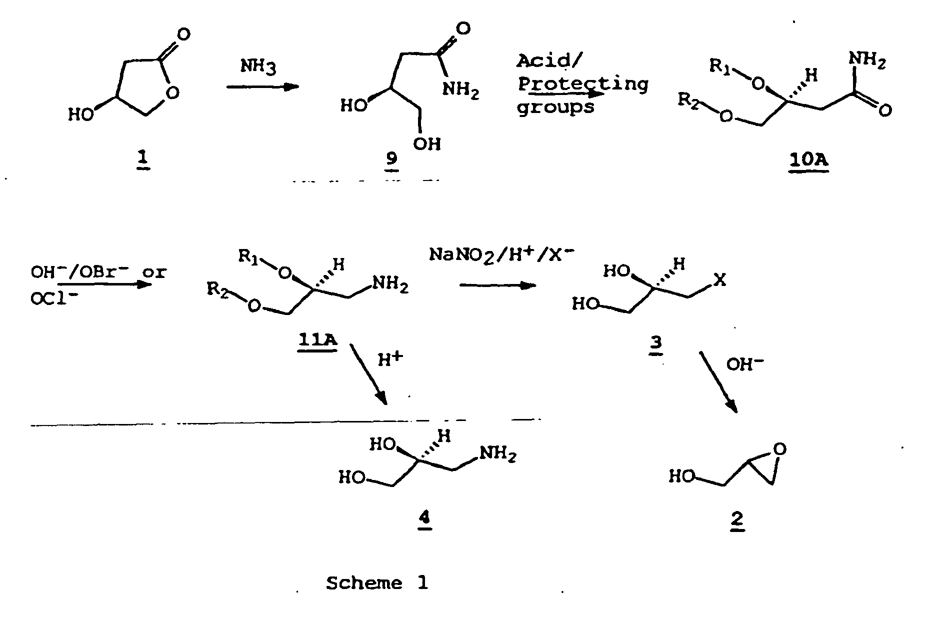 In Scheme 1, R1 and R2 are protecting groups which are acetals, and X is a halide selected from the group consisting of Cl, F, Br, and I. In the process 3-hydroxy-Y-lactone **(1)** is reacted in a reaction mixture with ammonia which produces the 1,2-dihydroxybutyramide **(2).** To effect the transformation of the 4-carbon (S)-3-hydroxy-γ-butyrolactone **(1)** to the 3-carbon compounds, it first has to be converted to a protected dihydroxybutyramide (**10A**). The butyramide is then converted to a protected 3-carbon amine **(11A)** by treatment with hypohalite ion in the presence of hydroxide ion. The protected amine is then deprotected with an acid to give **4** or converted to a halo diol such as **3** by treatment with nitrous acid and halide ion. There are several established methods for converting **3** to the epoxyalcohol **2.**

The important step in obtaining **11A** is a 1-carbon chain descension step in which the 4-carbon protected dihydroxybutryamide (**10A**) is stereospecifically and quantitatively converted to the pure 3-carbon primary amine **(11A)** via a Hoffman rearrangement reaction on the protected amide **(10A).** In a Hoffman rearrangement reaction, primary amides react with OCl⁻ or OBr⁻ in the presence of a strong base to form amines with the loss of the carbonyl carbon atom. However, such a reaction on a γ-hydroxyamide normally fails because of participation by the alcohol function to form a lactone. One important aspect of this invention is that participation of the alcohol functions can be avoided by tying up the interfering alcohol functions with blocking or protecting groups. The 3 and 4 hydroxyl groups of the dihydroxybutyramide **(9)** can be protected with any combination of protecting groups which includes but is not limited to the constituents of the group consisting of alkyloxy, aryloxy, acyloxy, halo, sulfonyloxy, sulfate, phosphate or saccharide. In particular, the groups may be any combination of alkylidene, arylidene or acylidene groups which includes such acetals such as propylidene, benzylidene, ethylidene and methylidene. In a preferred embodiment, the protecting group is a geminal dimethoxy-acetal such as 2,2-dimethoxypropane which forms a cyclic acetal with the 3 and 4 hydroxyl groups of the dihydroxybutyramide (2) to form the protected dihydroxybutyramide (**10A**).

Scheme 2 shows the preferred synthesis of 3-amino-1,2-dihydroxypropane isopropylidene acetal (**11**) and 3-carbon chiral intermediates.

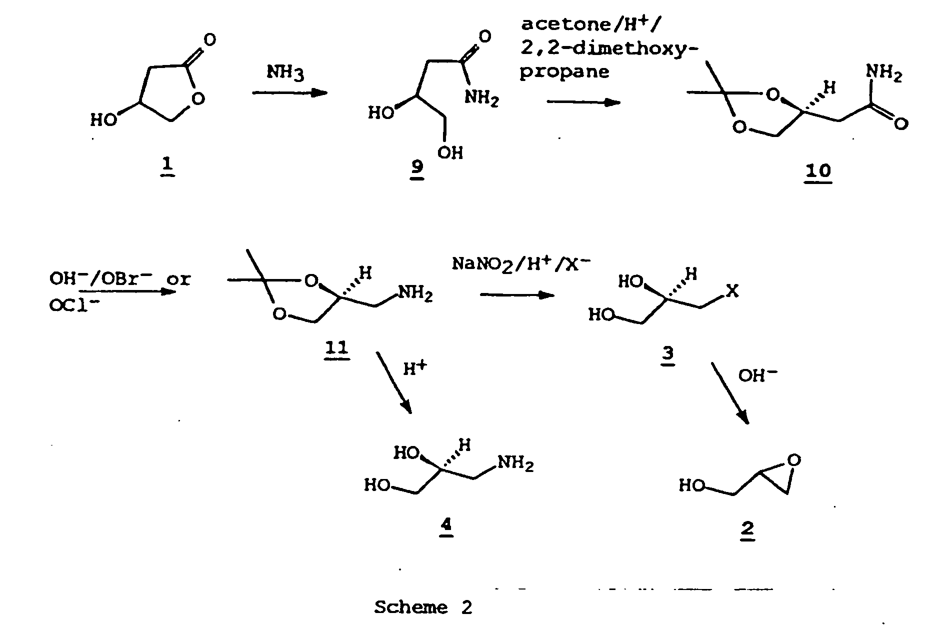

In Scheme 2, X is a halogen such as chlorine, iodine, fluorine or bromine. In the process, 3-hydroxy-γ-butyrolactone **(1)** is converted to 3,4-dihydroxybutyramide (**9**) in a reaction with ammonium hydroxide at room temperature. The reaction of the 3-hydroxy-γ-butyrolactone with ammonia in step (a) is preferably in aqueous solution at temperatures between -70°C and 100°C. The ammonia is preferably between 1 and 4 molar equivalents, optimally 1.2. The water is removed preferably at reduced pressures (10 to 30 mm of Hg) at 30° to 50°C. After removal of water, **9** is converted to the protected butyramide **(10)** in a reaction mixture containing a protecting group such as 2,2-dimethoxypropane, and an acid in a solvent such as acetone. In particular, **9** is reacted with acetone and dimethoxypropane in the presence of a strong acid (sulfuric, hydrochloric, phosphoric, toluene sulfonic acid) at 15° to 70°C for at least 30 minutes and then held at 20 to 25°C for about 6 to 12 hours. Silver oxide is added to eliminate the acid. Any compatible neutralizing agent can be used. The mixture is filtered and then it is concentrated to dryness to produce 2,3-dihydroxybutyramide isopropylidene acetal **10**. This reaction is quantitative and produces the protected butyramide (**10)** which can be crystallized upon concentration to dryness. A Hoffman rearrangement reaction completely converts **10** to 3-amino-1,2-dihydroxypropane isopropylidene acetal **(11).** In particular, **10** is reacted with a hypohalite (Cl, I, or Br) in the presence of a base (preferably an alkali metal hydroxide such as sodium hydroxide, potassium hydroxide) with heating at a temperature between 40 and 70°C to produce 3-amino-1,2-dihydroxy isopropylidene acetal **11** which is a key intermediate in the preparation of other compounds, particularly if the product is chiral. For example, **11** can be converted to 1-halo-2,3-dihydroxypropane halide **(3)** in a reaction containing an acid and the halide which can then be converted to an epoxyalcohol **(2)** in a reaction containing a base. Alternatively, reacting **11** with an acid will convert the protected amide to 3-amino-1,2-dihydroxypropane **(4).**

The bromodiol ((R)-1-bromo-2,3-dihydroxypropane) can be readily converted to (R)-glycidol by treatment with silver oxide at room temperature in a polar aprotic solvent such as dimethylformamide or dimethylsulfoxide. The dihydroxyamide **9** can be protected with any acetal function including benzylidene, ethylidene, methylidene and propylidene. In addition, it may be protected by conversion to a cyclic carbonate by treatment with reagents such as phosgene, ethylchloroformate or the corresponding acyl imidazole.

In the present invention, the carbonyl carbon of **10,** the protected form of **9,** which is formed from the lactone **(1)** is removed via a Hoffman rearrangement reaction . In preparing 3-carbon intermediates, the present invention has several advantages over the prior art. The major advantage is that **1** is readily available in high optical and chemical purity and the reagents employed in the 1-carbon descension are cheap and safe to handle. There are no environmentally undesirable materials such as heavy metal ions involved as in some prior art processes (Byun, Y.-S., et al., J. Org. Chem. 59:668-671 (1994)). The optical purity of the product is high unlike in some prior art methods (Byun, H.-S., et al., J. Org. Chem. 59:668-671 (1994); Bednarski, M. D., et al., J. Am. Chem. Soc. 109:1283-1285 (1987); Fuganti, C., et al., Tetrahedron 44:2575-2582 (1988); and Chen, J., et al., Tetrahedron Lett., 34:7663-7666 (1993)). Furthermore, unlike in the prior art, recovery is simple with just a solvent extraction required. Therefore, the instant process provides a process the synthesis of chiral 3-carbon synthons from chiral 3-hydroxy-y-butyrolactone **1** which is virtually quantitative and avoids the use of expensive transition metal catalysts or enzymes as are used in the prior art. Furthermore, the process is inexpensive to perform and uses renewable natural resources such as starch, lactose, maltodextrins and other readily available carbohydrate feedstock.

The following examples are intended to promote a further understanding of the present invention.

### EXAMPLE 1

This Example shows the preparation of (S)-3-amino-1,2-dihydroxypropane isopropylidene acetal **11** from (S)-3-hydroxy-γ-butyrolactone **1.**

(S)-3-hydroxy-γ-butyrolactone (51 g, 0.5 mol) was converted to the amide **9** by treatment at room temperature for 14 hours with 110 ml of 30% ammonium hydroxide (0.85 mol). The solution was then concentrated to a syrup at ~50°C under reduced pressure until no more water could be removed. Acetone (500 mL) and 2,2-dimethoxypropane (104 g, 1 mol) was added. Sulfuric acid (2 mL) was then added and the mixture protected from moisture with a calcium chloride drying tube, heated at 60°C for 30 minutes and stirred at room temperature for 12 hours. Silver oxide (20 g) was added and the mixture stirred for 1 hour. Methanol (200 ml) was then added and the mixture filtered and concentrated to dryness. The amide (**10**) crystallized on concentrating and was used directly in the next step. Conversion was essentially quantitative. A small amount when recrystallized from acetone gave white crystals mp, 98-100°C. [α]₅₈₉ = -15.4 (CHCl₃, c=1), ¹H-NMR (CDCl₃, 300 MHZ) δ 6.10 (s, 1H), 5.65 (s, 1H), 4.43 (m, 1H), 4.14 (dd, 1H, J=8.1 and 6.3 Hz) 3.63 (dd, 1H, J=8.1 and 6.8 Hz) 2.55 (dd, 1H, J=15.3 and 7.5 Hz), 2.46 (dd, 1H, J=15.3 and 4.8 Hz), 1.42 (s, 3H), 1.35 (s, 3H) ¹³C-NMR (CDCl₃, 75 MHZ) δ 172.86, 109.50, 72.21, 69.05, 40.07, 26.90, 25.50.

The amide (**10**) (1.59 g 0.01 mol) was treated with 10 to 12% sodium hypochlorite solution (10 ml) and the mixture stirred until all of the solid had dissolved (-5 mins). Sodium hydroxide (1.59 g dissolved in 10 ml water) was added to the mixture and the solution was warmed to 50-60°C and then kept there for 24 hours by which time conversion to amine **11** completed. ¹H-NMR spectroscopy indicated 100% conversion of **10** to **11**. The amine **11** was isolated by extraction of the mixture with ether as a light yellow liquid which upon standing gave colorless crystals mp 54-56°C. The yield was 1.11 g (85%). The amine **11** has previously been reported to be liquid, bp 62-65°C, 15 torr (Danklmaier, J., Hoenig, H., Liebigs Ann. Chem. 1149-1154 (1988)) probably because it had not been isolated in as pure a state as reported herein. [α]₅₈₉ = +0.9 (CHCl₃, c=1), ¹H-NMR (CDCl₃, 300 MHZ) δ 4.13 (m, 1H), 4.00 (dd, 1H, J=8.1 and 6.6 Hz), 3.67 (dd, 1H, J=8.1 and 6.3 Hz), 2.85 (dd, 1H, J=13.2 and 4.2 Hz), 2.78 (dd, 1H, J=13.2 and 6.0 Hz), 1.40 (s, 3H), 1.34 (s, 3H), 1.31 (s, 2H). ¹³C-NMR (CDCl₃ 75 MHZ) δ 109.10, 66.90, 44.71, 26.81, 25.31.

### EXAMPLE 2

This Example demonstrates the conversion of (S)-3-amino-1,2-dihydroxypropane **11** to (R)-1-bromo-2,3-dihydroxypropane **3**.

(S)-3-Amino-1,2-dihydroxypropane isopropylidene acetal **(11)** (0.10 g) was dissolved in 4.5 ml water. Hydrogen bromide (hydrobromic acid) solution (0.5 ml, 47% aqueous solution) and 0.52 g of sodium bromide were added to the solution which was then cooled to 0°C. Sodium nitrite (0.70 g) was added to the mixture and it was stirred at room temperature for 20 hours. A sample was tested by NMR spectroscopy which indicated complete conversion of the aminodiol to the bromodiol. The mixture was neutralized by sodium bicarbonate, then most of the water was removed by rotary evaporation and the residue was extracted in chloroform. The chloroform phase was dried over sodium sulfate; removal of the solvent gave the bromodiol **3** as a yellow liquid. The yield was 0.095 g (80%). [α]₅₈₉ = -4.00 (CHCl₃, c=1), ¹H-NMR (CDCl₃, 300 MHZ) δ 3.93 (m, 1H), 3.77 (dd, 1H, J=11.4 and 3.6 Hz), 3.66 (dd, 1H, J=11.4 and 6.0 Hz), 3.85-3.46 (m, 2H). 13C-NMR (CDCl₃ 75 MHZ) δ 71.44, 64.27, 34.62.

### EXAMPLE 3

This Example shows the conversion of (S)-3-amino-1,2-dihydroxypropane isopropylidene acetal **11** to (S)-1-chloro-2,3-dihydroxypropane **3**. This was done as described for the preparation of the corresponding bromo- compound above except that the sodium bromide was replaced by sodium chloride and hydrochloric acid was used instead of hydrobromic acid.

(S)-3-Amino-1,2-dihydroxypropane isopropylidene acetal **(11)** (0.10 g) was dissolved in 4.5 ml water. Hydrochloric acid solution (0.5 ml, 37% aqueous solution) and 0.52 g of sodium bromide chloride were added to the solution which was then cooled to 0°C. Sodium nitrite (0.70 g) was added to the mixture and it was stirred at room temperature for 20 hours. A sample was tested by NMR spectroscopy which indicated complete conversion of the aminodiol to the chlorodiol. The mixture was neutralized by sodium bicarbonate, then most of the water was removed by rotary evaporation and the residue was extracted in chloroform. The chloroform phase was dried over sodium sulfate; removal of the solvent gave the chlorodiol **3.**

### EXAMPLE 4

This Example shows the conversion of (S)-3-amino-1,2-dihydroxypropane isopropylidene acetal **11** to (S)-3-amino-1,2-dihydroxypropane **4**. This conversion was readily effected by treatment of the acetal **(11)** with 1.1 equivalents of a mineral acid in water followed by the removal of the solvent by rotary evaporation. This yielded the corresponding salt.

### EXAMPLE 5

This example shows the preparation of (R)-glycidol **(2)** from (R)-1-bromo-2,3-dihydroxypropane **(3).** This conversion was readily effected by treatment of the bromodiol with a base in water which yielded the epoxyalcohol **(2).**

### EXAMPLE 6

This example shows the preparation of (R)-3-Chloro-1,2-propanediol **(12)** from (S)-3-amino-1,2-dihydroxypropane isopropylidene acetal **(11).** The amine **11** 2.62 g (0.02 mol) was dissolved in 10 ml water. Sodium chloride 8.78 g (0.15 mol) was added along with concentrated hydrochloric acid (37%) 20 ml (0.2 mol) diluted with 10 ml water. Sodium nitirte 10.4 g (0.15) was then added over a period of 10 minutes. The mixture was then stirred for 24 hours after which time an analysis of the reaction by NMR spectroscopy indicated complete conversion to the chlorodiol. The mixture was then concentrated to dryness and the product was extracted with chloroform 3 or 4 times. The extracts were combined and dried with sodium sulfate. Removal of the solvent gave the chlorodiol **12** as a light yellow liquid 1.81 g (82%). [α]₅₈₉ = -7.2 (H₂O, c=5), ¹H-NMR (D₂O, 300 MHZ) δppm 3.86 (m, 1H), 3.68-3.48 (m, 4H). ¹³C-NMR (CDCl₃ 75 MHZ) δppm 71.7, 63.6, 45.8. All products were >99.5% optically pure by chiral G.C.

## Claims

1. A process for preparing a protected 3-amino-1,2-dihydroxypropane acetal in a Hoffman rearrangement reaction mixture, having the formula (1): 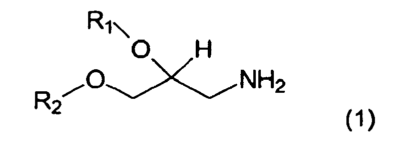 wherein R₁ and R₂ are protecting groups which can be combined
which comprises
reacting a protected 1,2-dihydroxybutryamide in a reaction mixture, having the formula (2): 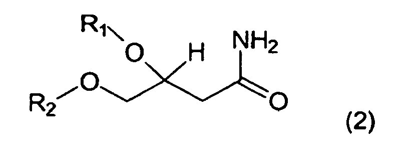 wherein R₁ and R₂ are the same protecting groups
with a hypohalite in the presence of a base in a solvent for the reaction mixture and recovering the protected 3-amino-1,2-dihydroxypropane acetal by solvent extraction.

2. The process according to claim 1, further comprising the step of reacting the compound of formula 1 with a halide source in the presence of an acid, thereby to form a compound of formula (3): 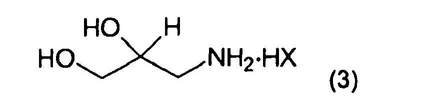 wherein X is a halogen, preferably Cl or Br.

3. The process according to claim 1, further comprising the step of reacting the compound of formula (1) with a halide source and a nitrite in the presence of an acid, thereby to form a compound of formula (4): 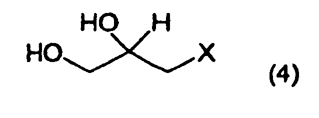 wherein X is a halogen, preferably Cl or Br.

4. The process according to claim 3, further comprising epoxidising the compound of formula (4) to form a compound of formula (5):

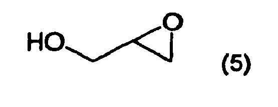

5. The process according to any preceding claim, wherein R₁ and R₂ are selected from the group consisting of acetal protecting groups, cyclic carbonate protecting groups, alkoxy protecting groups, aryloxy protecting groups, acyloxy protecting groups, halo protecting groups, sulfonyloxy protecting groups, sulphate protecting groups, phosphate protecting groups, saccharide protecting groups, and combinations thereof.

6. The process according to claim 5, wherein R₁ and R₂ represent an alkylidene, arylidene or acylidene acetal protecting group.

7. The process according to claim 6, wherein R₁ and R₂ represent a benzylidene, ethylidene, methylidene or propylidene acetal protecting group.

8. The process according to any preceding claim, wherein the hypohalite is hypochlorite or hypobromite.

## Patentansprüche

1. Verfahren für die Herstellung eines geschützten 3-Amino-1,2-dihydroxypropanacetals in einer Hoffman-Umlagerungsreaktionsmischung, mit der Formel (1): 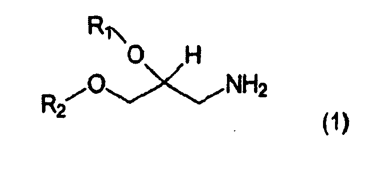 wobei R₁ und R₂ Schutzgruppen sind, die kombiniert sein können, umfassend:
Reagieren eines geschützten 1,2-Dihydroxybutyramids in einer Reaktionsmischung, mit der Formel (2): 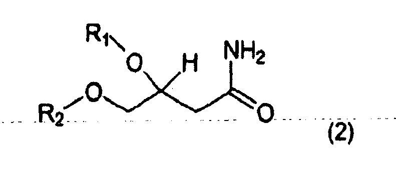 worin R₁ und R₂ die gleichen Schutzgruppen sind,
mit einem Hypohalit in der Gegenwart einer Base in einem Lösungsmittel für die Reaktionsmischung und Wiedergewinnen des geschützten 3-Amino-1,2-dihydroxypropanacetals durch Lösungsmittelextraktion.

2. Verfahren nach Anspruch 1, ferner umfassend den Schritt des Reagierens der Verbindung von Formel 1 mit einer Halogenidquelle in Gegenwart einer Säure, um **dadurch** eine Verbindung der Formel (3) zu bilden: 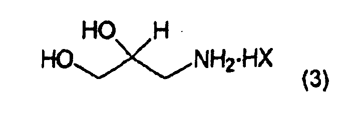 worin X ein Halogen, vorzugsweise CI oder Br ist.

3. Verfahren nach Anspruch 1, ferner umfassend den Schritt des Reagierens der Verbindung der Formel (1) mit einer Halogenidquelle und einem Nitrit in Gegenwart einer Säure, um **dadurch** eine Verbindung der Formel (4) zu bilden: 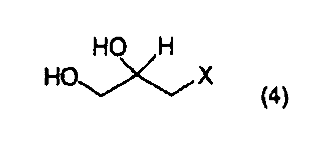 worin X ein Halogen, vorzugsweise Cl oder Br ist.

4. Verfahren nach Anspruch 3, ferner umfassend das Epoxidieren der Verbindung der Formel (4), um eine Verbindung der Formel (5) zu bilden:

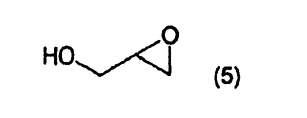

5. Verfahren nach einem der vorangehenden Ansprüche, wobei R₁ und R₂ ausgewählt sind aus der Gruppe bestehend aus Acetalschutzgruppen, cyclischen Carbonatschutzgruppen, Alkoxyschutzgruppen, Aryloxyschutzgruppen, Acyloxyschutzgruppen, Haloschutzgruppen, Sulfonyloxyschutzgruppen, Sulfatschutzgruppen, Phosphatschutzgruppen, Saccharidschutzgruppen und Kombinationen davon.

6. Verfahren nach Anspruch 5, worin R₁ und R₂ eine Alkyliden-, Aryliden- oder Acylidenacetalschutzgruppe bedeuten.

7. Verfahren nach Anspruch 6, worin R₁ und R₂ eine Benzyliden-, Ethyliden-, Methyliden- oder Propylidenacetalschutzgruppe bedeuten.

8. Verfahren nach einem der vorangehenden Ansprüche, wobei das Hypohalit Hypochlorit oder Hypobromit ist.

## Revendications

1. Procédé de préparation d'un 3-amino-1,2-dihydroxypropane acétal protégé dans un mélange réactionnel à réarrangement de Hoffman, ayant la formule (1) : 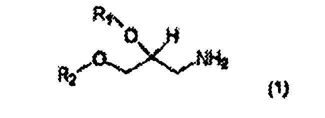 dans laquelle les groupes R₁ et R₂ sont des groupes protecteurs qui peuvent être combinés
qui comprend
la réaction d'un 1,2-dihydroxybutyramide protégé dans un mélange réactionnel, ayant la formule (2) : 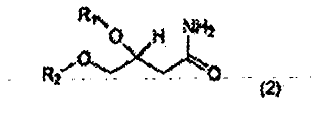 dans laquelle les groupes R₁ et R₂ sont les mêmes groupes protecteurs
avec une hypohalite en présence d'une base dans un solvant pour le mélange réactionnel et la récupération du 3-amino-1,2-dihydroxypropane acétal protégé par une extraction par solvant.

2. Procédé selon la revendication 1, comprenant en plus l'étape de mise en réaction du composé de formule 1 avec une source d'halogénure en présence d'un acide pour former ainsi un composé de formule (3) : 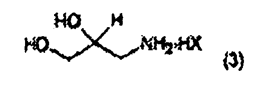 dans laquelle X est un halogène, de préférence Cl ou Br.

3. Procédé selon la revendication 1, comprenant en plus l'étape de mise en réaction du composé de formule (1) avec une source d'halogénure et un nitrite en présence d'un acide afin de former ainsi un composé de formule (4): 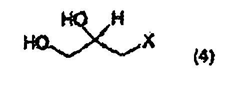 dans laquelle X est un halogène, de préférence C1 ou Br.

4. Procédé selon la revendication 3, comprenant en plus l'époxydation du composé de formule (4) afin de former un composé de formule (5):

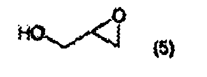

5. Procédé selon l'une quelconque des revendications précédentes, dans lequel R₁ et R₂ sont sélectionnés dans le groupe constitué des groupes protecteurs acétal, des groupes protecteurs de carbonate cyclique, des groupes protecteurs alcoxy, des groupes protecteurs aryloxy, des groupes protecteurs acyloxy, des groupes protecteurs halo, des groupes protecteurs sulfonyloxy, des groupes protecteurs de sulfate, des groupes protecteurs de phosphate, des groupes protecteurs de saccharide et des combinaisons de ceux-ci.

6. Procédé selon la revendication 5, dans lequel R₁ et R₂ représentent un groupe protecteur alkylidène, arylidène ou acylidène acétal.

7. Procédé selon la revendication 6, dans lequel R₁ et R₂ représentent un groupe protecteur benzylidène, éthylidène, méthylène ou propylidène acétal.

8. Procédé selon l'une quelconque des revendications précédentes, dans lequel l'hypohalite est l'hypochlorite ou l'hypobromite.
